Europäisches Patentamt

European Patent Office

Office européen des brevets

(11) Publication number: **0 109 847**
**B1**

## (12) EUROPEAN PATENT SPECIFICATION

(45) Date of publication of patent specification: **11.05.88**

(21) Application number: **83307085.7**

(22) Date of filing: **18.11.83**

(51) Int. Cl.⁴: **A 61 F 13/04**

(54) Fracture cast-braces and casts incorporating them.

(30) Priority: **18.11.82 GB 8232996**
**23.02.83 GB 8305034**
**05.05.83 GB 8312293**

(43) Date of publication of application:
**30.05.84 Bulletin 84/22**

(45) Publication of the grant of the patent:
**11.05.88 Bulletin 88/19**

(84) Designated Contracting States:
**DE FR IT NL SE**

(56) References cited:
**WO-A-82/02658**
**FR-A-2 477 409**
**US-A-3 613 674**
**US-A-3 958 569**
**US-A-4 312 335**

(73) Proprietor: **PROTECTAIR LIMITED**
**P.O. Box 4**
**Watlington Oxon OX9 5QQ (GB)**

(72) Inventor: **Young, David Ernest**
**Bowlers Piece 16 Couching Street**
**Watlington Oxon. OX9 5QQ (GB)**
Inventor: **Boyes, David Holmes**
**223 Woolton Road**
**Childwall Liverpool (GB)**

(74) Representative: **Thiemann, Peter Albert William**
**et al**
**LLOYD WISE, TREGEAR & CO. Norman House**
**105-109 Strand**
**London WC2R 0AE (GB)**

EP 0 109 847 B1

Courier Press, Leamington Spa, England.

## Description

This invention relates to fracture cast-braces or hinge-braces and to casts incorporating such braces.

White Plaster of Paris casts, such as full leg casts, or casts which are applied to other areas, where immobilisation of fractured bone, or bone required to fixate by virtue of elective surgery, is required are well known for ensuring that fractured ends of bone, once "set" or re-aligned so that they approximate closely, do not undergo relative motion or displacement.

There are, however, therapeutic consequences to the use of plaster casts on long bones, not to mention certain inherent disadvantages of Plaster of Paris itself. Problems can be particularly pronounced when full leg casts are used on the long bones of the leg.

Commonly, orthopaedic surgeons and plaster technicians apply the cast not only to the affected part of the limb, but also to the adjacent (proximal or distal) part, e.g. the full leg or full arm cast. This is to ensure that most of the compressive load is transmitted from points beyond to points before the fracture. Perhaps even more important is the prevention of rotation which, if unchecked, could cause further damage at the fracture site, perhaps culminating in non-union of it.

Casts also provide general mechanical protection and their very nature encourages the patient to "guard" the injury.

When the whole leg is immobilised in a plaster cast for, say, a mid-shaft fracture of the femur, restriction of movement may continue for several months until healing is complete. Joint stiffness, together with considerable wastage of muscle under the plaster is common. In the wake of this, there is considerable need for physiotherapy and rehabilitative services which constitute a drain on health-care resources. Disturbances of gait and delayed return to work, school or other occupation are all additional sequels. To a lesser extent, some of these consequences occur when the long bones of the arm are immobilised for a long period and it is worth mentioning the delay in return to car driving which is often associated with the extended application of a rigid cast.

Recognition and awareness of these problems has made orthopaedic surgeons and plaster technicians receptive to methods which enable the benefits of plaster casting to be had, but with a reduction in the undesirable side effects. A major contribution to this aim has been the concept of cast-bracing which is otherwise called hinge-bracing or fracture-bracing.

This concept involves the application of two casts to long bones either side of a joint, e.g. above and below the knee or above and below the elbow. The two casts are jointed at the time they are formed by hinges, carefully applied so that the hinge axis lies along the condylar axis of the joint, the top and bottom of each hinge being embedded in the plaster cast.

Since the 1960's, the gradual development of the concept of ambulatory care of lower extremity fractures has progressed to the point where cast-bracing is now almost universally approved and accepted. The hinged cast has the fundamental advantage of allowing early motion in a joint. Furthermore, in the case of distal femur and upper tibial fractures, there is the advantage of early ambulation in addition to maintaining motion of the knee. Rotation is controlled.

Cast-bracing allows partial weight bearing at the fracture itself which promotes healing and mobilises oedema fluid which would otherwise accumulate in the capsular and ligamentous structures of the joints. This is important since such accumulation leads to joint stiffness and temporary (sometimes permanent) loss of function.

The basic concept of cast-bracing or hinge-bracing is that immobilisation is unphysiologic while mobilisation is always physiologic. The solutions of the cast-brace are thus physiological solutions.

Cast-braces in common use fall into two main categories:

a. Monocentric hinges:
These are usually made of metal, such as steel, aluminium or aluminium alloy, and the upper and lower components are hinged together in a conventional manner about a single axis, the form of the actual hinge being of the bar and disc type. At the top and bottom of each component is a plate which is secured to the bar. This type of hinge allows no possibility of medial or lateral movement at the joint it is bracing. Elevation and depression of the distal part of the limb relative to the proximal part about a single axis is the only type of movement which this hinge allows for — hence the name "monocentric". Clearly very accurate positioning of both medial and lateral hinge axes on the elevation/depression axis of the joint is very important, otherwise these movements will be very difficult and torsional forces may be brought to bear on the affected limb. Fixation can only be correctly carried out by using a jig to ensure the centres of rotation of both hinges lie on the same axis and that they are in the same sagittal plane.

b. Polycentric hinges:
These hinges may be made of metal or plastics. In well known commercially available metal hinges there is employed a pair of hinge mounting brackets which hold the arms of the hinge in the same plane. The hinge arms have radial teeth, as in a cog, and these engage radially and pivotally in the region of the mid point between their respective hinge axes. Fixing means pass through holes in the arms and the brackets and also constitute hinging means. A number of modifications to this basic mechanism allow the hinge to be locked in various positions. Cast brace hinges are fitted with head-plates at the extreme ends of the hinge arms. The object of these is to fix the hinge firmly within the casts.

Polycentric hinges made of plastics usually provide hinging by virtue of the property of maintained flexibility during repeated flexing and relaxing of modern polymer plastics including high molecular weight polyethylene. This is enhanced by the shaping of a single hinge component into broad convolutions parallel to the axis of elevation/depression of the limb and at right angles to the axis of the limb itself. Flexure of the hinge component may occur at any of these convolutions — hence the hinge is termed polycentric.

It is a feature of this type of hinge that on flexure, reactive forces build up within it, so that the musculature of the limb derives benefit from isometric exercises against the hinge. During walking, this effect also manifests as a gentle "throwing forward" of the leg which assists the patient in assuming a more normal gait despite having to wear the plaster cast. Because repeated flexure of plastic will eventually lead to fatigue of the material, plastic cast-braces are intended to be discarded after single use. At the top and bottom of each hinge are head-plates very similar (sometimes identical) to those used on monocentric bracing hinges. They are usually pressed from thin plastic sheet into rectangular plates with rounded corners, typically they have four holes about 1 cm in diameter, pressed through them in a regular pattern. These plates are usually rivetted to the inner aspect of the hinge which has a flattened end formed and drilled for the purpose.

Such a construction is illustrated in International Application No. WO 82/02658 which describes an improved orthotic device for use in knee stabilisers and modified cast braces comprising a head plate having inner and outer surfaces with respect to a limb to which it is to be applied secured at the end of a hinge portion. The hinge portion described in the specification has a rigid centre support member having an upper and lower end and upper and lower flanges non-slidably pivotally hinged to the upper and lower ends respectively of said centre support member. Preferably attachment wings or head-plates are each separately and individually secured to the upper and lower flanges. The attachment wings are in the form of rectangular planar sheet elements curved to provide inner and outer surfaces with respect to a limb to which the cast brace is to be applied and are attached to the ends of said flanges. Alternatively, the flanges may be flared to simulate attachment wings. The attachment wings are formed with a regular pattern of holes.

It is accepted as axiomatic by the professions who use and fit hinge-braces that there must be good contact between the Plaster of Paris and the plates on the hinge.

The holes in the plate enable wet plaster bandage to be pushed into them, so making contact with the bandage previously applied and improving lamination and bonding-in of the hinge plate. Even so, it is not always possible to achieve good lamination because the hinge component rises significantly from the plate and it is very difficult to eliminate dead space under the portion of the bandage adjacent to the hinge.

Plaster of Paris has a number of inherent disadvantages as a material for making casts and these disadvantages are well recognised. The setting time can be as much as 36 hours and patients may have to be hospitalised during this period. These casts are heavy and uncomfortable for the patient to wear. The casts are opaque to X-rays, hence the progress towards healing cannot be monitored through the cast when Plaster of Paris is used.

These factors were probably the main motivation behind the development of the modern casting materials, including those marketed under the Trade Marks, "Baycast", "Orthoplast", "Hexvelite", "Zoroc", "Scotchcast" and "Litecast".

Most of these materials set quickly to load-bearing (in some cases as little as 20 minutes) and are radio-apparent as well as being very much lighter than Plaster of Paris.

"Baycast" for instance, is a polyurethane resin system impregnated onto a woven fabric with threads measured at 0.5 mm across and 0.25 mm down, enclosing interstices of 1.00 mm by 0.5 mm. The fabric usually has very little elasticity.

The resin curing period is accelerated by water, moisture in the air being usually sufficient to promote initial setting in a few minutes. The cured bandage is thin but very strong in the same plane as the weave. Strength increases rapidly as successive layers are built up, but it is essential, as with all casting materials, that good lamination is achieved between layers. This means there must be continuous contact between them.

Unfortunately, the failure to achieve excellent lamination when using the new casting materials in conjunction with hinge-braces has limited acceptance of this combination. Despite instructions by the manufacturers to bind the braces into the cast well down onto the hinged component past the head-plate, de-lamination and loosening of the braces is quite common. This is a potentially disastrous situation if the fractured limb suddenly becomes loaded.

There are several reasons for this problem of hinge/bond failure. Firstly, the fabric or base material of the new casting materials is not generally amenable to the "push-through" approach to the holes in the head-plate of the brace which is used with the soft bandage of the Plaster of Paris casting technique. Consequently, the holes simply represent lost bonding area.

Pressing the bandage against the holes in the head-plate to form a 'dimple' which engages with the holes to some extent on setting, is probably acceptable on the lateral aspect of the limb but on the medial aspect, there is an associated risk of nerve compression. This leads many users to eschew the practice. In any case, there is always a risk of using too much pressure and deforming the cast inwards. This could cause impingement on the limb and consequent skin abrasion.

Secondly, the significantly raised area of the head-plate at and adjacent to the portion thereof where the hinge part joins the head-plate causes a dead space under the bandage. The dead space significantly reduces the surface area over which the layers of casting material contact the head-plate.

The use of rivets to secure the head-plate to the brace intensifies the problem since the rivets form excrescences on both faces, which, though small, still "lift" the bandage slightly away from the faces.

The combined effect of the holes in the plate and the dead space close to the hinge and around the rivets probably represent together loss of over 40% of the bonding area on the outside face and about 20% on the inner face. It should be borne in mind that these bandage or bracing materials have little comformability to tight radius curves but this is precisely what current hinge/head-plate designs impose.

Finally, it must be mentioned that virtually no bonding occurs between head pieces made of smooth polyethylene and the modern polyester or polyurethane casting materials.

It is hardly surprising then that there have been less than totally satisfactory results with modern casting materials in hinge-bracing when the underlying design of current hinge-braces is considered.

It is an object of the present invention to provide an improved hinge-brace for fracture casts, and one which is particularly suitable for use with modern resin casting materials.

According to one aspect of the present invention there is provided a hinge-brace for a fracture cast comprising a head-plate for incorporation in a fracture cast, the head-plate having inner and outer surfaces with respect to a limb to which it is to be applied, and being secured to one end of a hinge portion, characterised in that the hinge brace comprises at least one head-plate, the central region of the, or each of which is thicker than the edge regions, the central region or an extension of the head-plate being formed with a recess and the end of the hinge portion being shaped in a manner complementary to said recess and being a close fit therein, and in that the inner and outer surfaces of the hinge-brace which are intended to lie within the cast are bounded by substantially smooth concave and convex surfaces respectively, whereby when the head-plate is embedded in casting material, the formation of dead spaces in the region where the hinge portion joins the head-plate is substantially eliminated.

Preferably the hinge portion and head-plate are so shaped and secured to one another that the outer surface of the head-plate and a corresponding outer surface of the hinge portion, are substantially coplanar and that the inner surface of the head-plate and a corresponding inner surface of the hinge portion are likewise substantially coplanar, said outer and inner surfaces being those which are intended to lie within the cast.

The hinge portion may be a polycentric hinge or monocentric hinge and may be made of plastics or metal as discussed above. The brace preferably has a head-plate at each end of the hinge portion and the thickness of the central region of the head-plate is equal to that of the end of the hinge portion. If desired, the or each head-plate may be formed with an extension from the thicker central region for receiving the end of a hinge portion, the extension having at least one surface coplanar with a corresponding surface of the head-plate and intended to lie within the cast. Alternatively, the central region of the head-plate or the extension therefrom is formed with a recess and the end of the hinge portion is shaped in a manner complementary to said recess and is a close fit therein. The recess may be a slot formed in the central region or an open recess in one of the surfaces with the end of the hinge portion rebated to fit closely in the recess and provide the substantially coplanar surfaces. As the head-plates have thicker central regions and thinner edge regions they are substantially crescent shaped in cross-section and are preferably of arcuate crescent shape. At their thickest points the central regions of the head-plates are preferably about 25% to 30% thinner than riveted-hinge-to-head-plate thickness in conventional designs. At their thinnest points (along the edges) they have a thickness similar to existing braces. In one embodiment, the inner face of the head-plate has a moulded open recess exactly matching the end of the hinge portion except for two stakes which engage holes in the hinge portion. These stakes form spin-welds when pressed firmly with a suitable spinning glass or metal die. The excrescence on the inner hinge is minimal and less than a rivet.

Experiments on a "standard leg" and "standard arm" made from lengths of 6" (15.2 cm) and 4" (10.2 cm) heavy duty plastic soil-pipe respectively, show that the present brace provides much better laminating surfaces than previously proposed braces as measured by increased mechanical bond strength and by other tests. Performance may be enhanced by the provision in the head-plates of short narrow-angle splines which may be incorporated to face various impressed or reactive force directions.

It is possible to use the present hinge brace with Plaster of Paris since with this material, too, lamination is improved.

In order to enable the invention to be more readily understood, reference will now be made to the accompanying drawings, which illustrate diagrammatically and by way of example some embodiments thereof and in which:—

Figure 1 is a plan view of a hinge-brace showing various modifications;

Figure 2 is a cross-section along the line II—II in Figure 1 before assembly of parts;

Figure 3 is a cross-section along the line II—II in Figure 1 after assembly of parts;

Figure 4 is a view along the line V—V in Figure 1;

Figure 5 is a view similar to Figure 3 of a further modification;

Figure 6 is a view of a further hinge-brace shown partly in top plan view and partly in under plan view, and

Figure 7 is a cross-section along the line VII—VII in Figure 6;

Figures 8 and 9 are respectively a perspective view from above and a perspective view from below of a head-plate for a hinge brace;

Figures 10 and 11 are respectively a perspective view from above and a perspective view from below of another head-plate for a hinge brace;

Figure 12 is a side view of a bracing hinge for a lateral brace, and

Figure 13 is a side view of a bracing hinge for a medial brace.

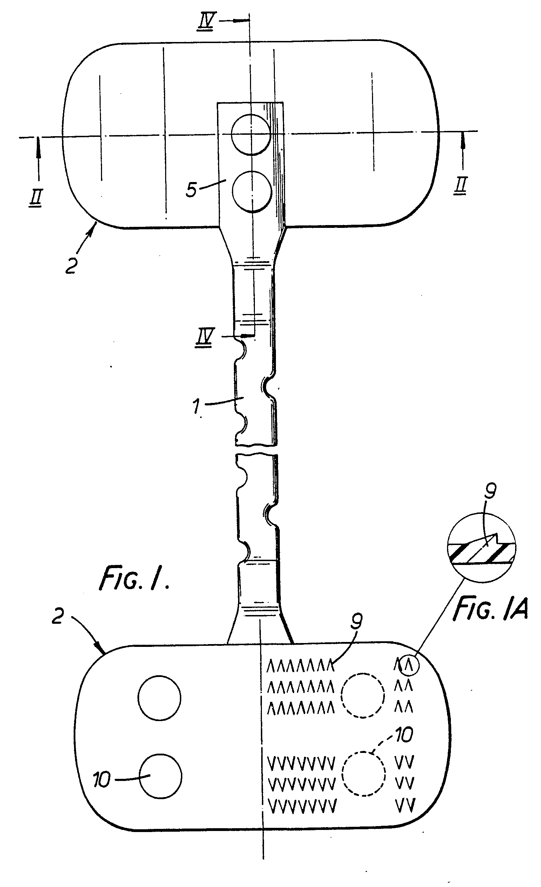

Referring now to Figure 1, there is shown a hinge-brace for a fracture cast. The brace comprises a polycentric hinge portion 1 which may for example be of plastics, such as polyethylene or nylon, and which may be moulded or formed from polyethylene bar. At each end, the hinge portion 1 is secured to a head-plate 2 and various head-plate modifications and methods of securing are shown in the drawings.

The bottom half of Figure 1 shows a head-plate 2, integrally moulded with the hinge portion 1 while the upper half of Figure 1 shows a separately made head-plate subsequently secured to the hinge portion.

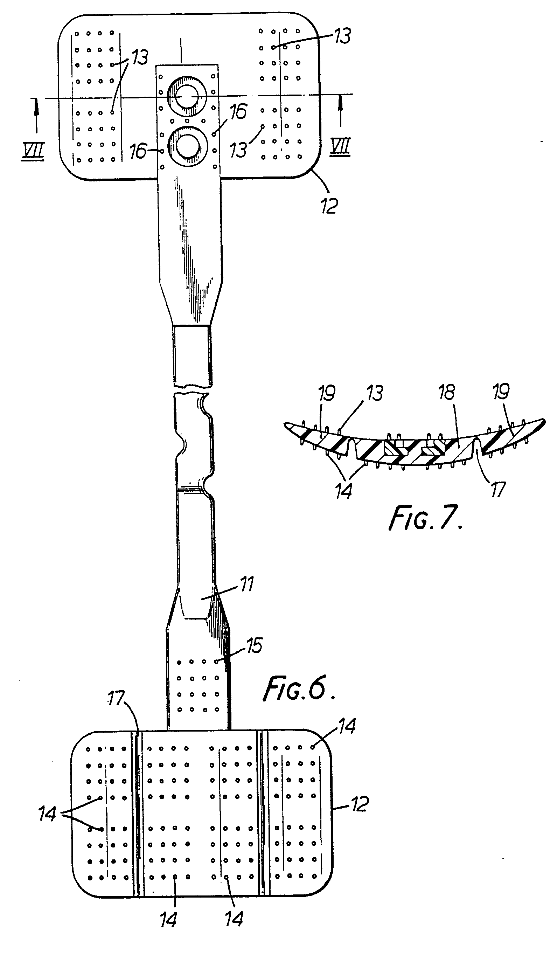

The hinge portion 1 is of generally conventional shape and may have its major portion displaced out of the plane of the paper to prevent the brace, when in use, from rubbing against the condyles of a joint (c.f. Figure 4).

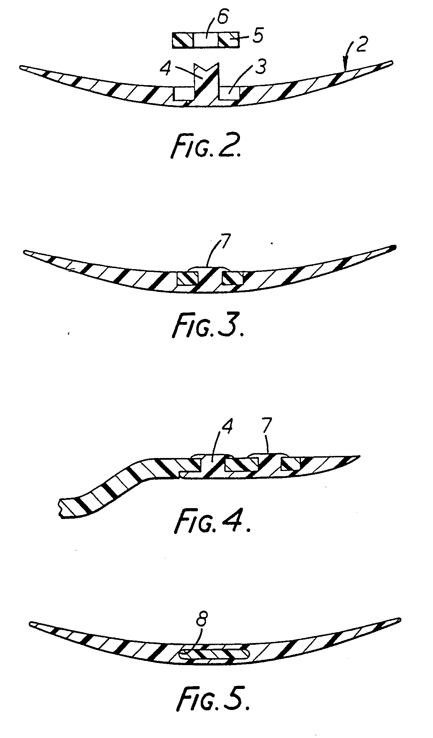

The head-plate 2, however, differs considerably from the head-plates of previously used hinge braces. As clearly shown in Figures 2, 3 and 5, the head-plate 2 is arcuate crescent-shaped in cross-section being thicker in its central region and thinner at the edges. Furthermore, the head-plate and hinge portion are secured together in a substantially flush manner so as substantially to avoid the possibility of a dead space forming when the brace is used. That is to say, an outer surface of the head-plate and a corresponding outer surface of a hinge portion are substantially coplanar as are an inner surface of the head-plate and a corresponding inner surface of the hinge portion.

The head-plate shown in Figures 2 to 4 is formed with a recess 3 in which are formed two upstanding stakes or stubs 4. The end 5 of the hinge portion 1 is formed with two holes 6 to receive the stakes 4 and has a thickness substantially equal to the depth of the recess so that, as shown in Figures 3 and 4, the end 5 of the hinge portion can be received in a substantially flush manner in the recess 3. The free ends of the stakes 4 are then compressed in a press using a spinning die which both swages and welds them down onto and partly into the hinge portion as shown at 7, thereby securing the hinge portion to the head-plate.

In the modification shown in Figure 5, the head-plate is formed with a central hole 8 to receive an appropriately shaped end of the hinge portion 1. If desired, adhesive or glue may be used to reinforce the connection between the head-plate and the hinge portion. Other forms of fixing the head-plate to the hinge portion are possible, including rivets, snap-on fixings, or adhesive fixings, provided that in each case the substantially flush fitting of the two parts is achieved.

The use of a hinge brace as just described is substantially the same as that of previously proposed braces. However, the arcuate cross-section of the head-plate provides close conformation with a limb and this conformation, together with the elimination of dead spaces due to the flush connection of the head-plates and hinge portion, ensure good lamination of the head-plates using modern resin casting materials.

In order to improve the lamination, the head-plates may be roughened or formed with teeth or splines 9 as shown at the bottom right hand side of Figure 1 and in Figure 1A. The teeth or splines 9 may be formed on one or both sides of the head-plate and may point in any direction, or, as shown, some may point in one direction and some in another.

The head-plates may be formed with holes 10 as is conventional but in order to enhance bonding and to reinforce the benefits of using a curved and shaped head-plate, circular pieces of the casting material may be provided with the brace to fill the holes and link the underlying and overlying layers of casting fabric material.

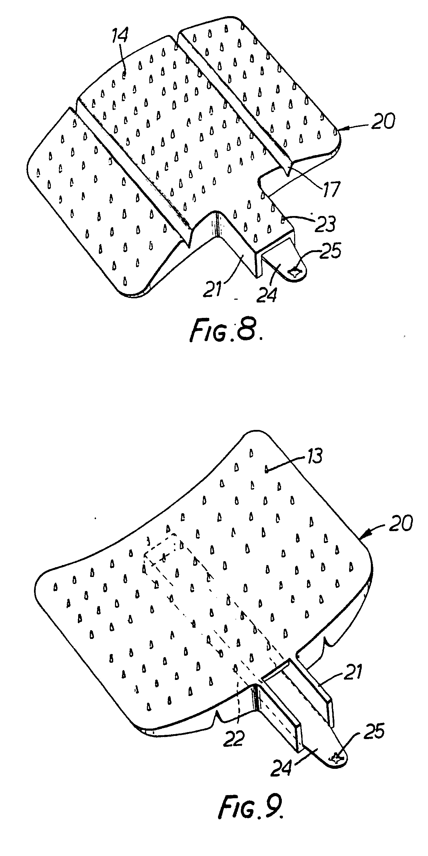

Referring now to Figures 6 and 7, there is shown another hinge-brace for a fracture cast, the top half of Figure 6 being an under plan view and the lower half a top plan view of a head-plate 12 each of which is secured to a polycentric hinge portion 11 in a manner similar to that discussed with reference to Figures 2 to 4. The inner or under surface of each head-plate 12 is formed with upstanding prongs 13 which are shown arranged in groups and the outer or upper surface of each head-plate is formed with upstanding prongs 14 which are shown also arranged in groups. As seen in Figure 7, the prongs 13 are shorter than the prongs 14. It will be seen that the polycentric hinge portion 11 is also formed at its ends with prongs 15 on the outer surface and 16 on the inner surface with the prongs 16 being shorter than the prongs 15. As shown in Figure 7, each head-plate 12 is formed with two grooves 17 running parallel to the portion 11 and sub-dividing the head-plate into a central portion 18 and two wing portions 19.

The head-plates of the hinge brace shown in Figures 6 and 7 are preferably made of suitable plastics, such as high density polyethylene or polypropylene so that the wing portions 19 can be bent relative to the central portion 18 without breaking whereby the head-plate can be closely conformed to limbs of even small dimensions. The hinge portion is conveniently made of nylon.

Finally, the surfaces of the head-plates and the ends of the hinge portion 11 have been roughened as by spark erosion or the like so that the hinge-brace does not present a smooth surface to the casting material.

As indicated above, several factors are important in maintaining a close and immobile relationship between a hinge-brace and the case in which it is incorporated. The first of these is to design and shape the hinge-brace in such a way as to reduce to a minimum the dead space which occurs with prior art braces. The second is to ensure good contact between the brace and the casting material as by roughening the surface of those parts of the brace which are to be embedded in the casting material. This can be effected by the provision of the teeth and splines 9 or by the prongs 13, 14, 15 and 16 which can engage through the weave of the casting material and effect sound mechanical capture of the casting material on to the hinge brace. The third factor is to ensure that, so far as possible, relative motion and distortion between the components of the hinge brace within the cast is eliminated. This may be effected by ensuring that the hinge portion fits tightly into the head-plate without play. In this connection it can be shown that the presence of a dead space in the region where the hinge portion is joined to the head-plate provides good conditions for leverage of the hinge portion relative to the head-plate leading to distortion of the connection, loosening of the connection and even failure. It will thus be appreciated that the first and third factors listed above are to some extent interdependent.

It will be appreciated that with the embodiment of the present hinge-brace shown in Figures 6 and 7, the grooves 17 represent dead spaces which, as indicated above, are to be avoided. However, these dead spaces are remote from the junction of the hinge portion and the head-plate and can, therefore, not contribute to relative motion and distortion between the hinge portion and the head-plate. Furthermore, as there are prongs 14 on each side of a groove 17 there is good mechanical capture of the casting material across the groove so that the presence of the grooves has the minimum deleterious effect on the hinge-brace while at the same time providing the advantage that by bending along the grooves, the head-plate can be conformed to a limb to be braced so as to eliminate the dead space that would otherwise exist between the wing portions 19 of the head-plate and the limb.

In the hinge brace shown in Figures 6 and 7 the parts of the hinge portion 11 that will lie within the finished cast are provided with prongs 15 and 16 and have been roughened so as to promote capture within the cast. This means that when the cast is removed from the patient and discarded the hinge brace is also discarded.

Figures 8 to 11 show two head-plates for hinge braces which can be used with re-usable hinge portions, especially metal monocentric or bicentric hinge portions, where it would be unduly expensive or impracticable to provide the hinge portion with prongs similar to the prongs 15 and 16.

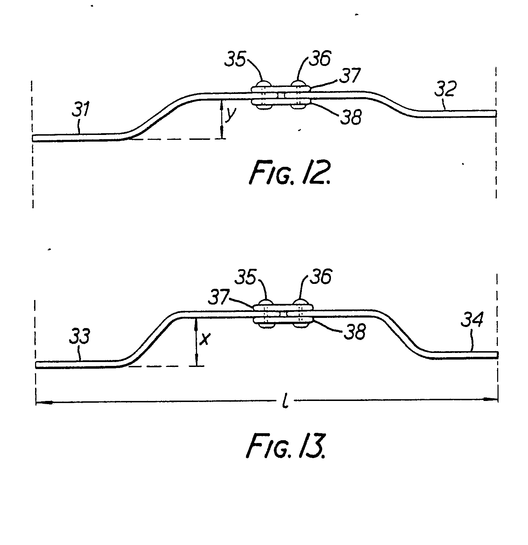

The head-plate 20 shown in Figures 8 and 9 is similar to the head-plate 12 in Figures 6 and 7 and is formed with the inner and outer prongs 13 and 14 and grooves 17. However, the head-plate 20 is formed with an extension 21 which is substantially U-shaped in cross-section and is formed with a hole 22 in the thicker central region, the trough of the extension 21 and the hole 22 serving to receive the end of a metal bar forming part of a monocentric, bicentric or polycentric hinge portion such as that described hereinafter with reference to Figures 12 and 13. The outer surface of the extension 21 is formed with prongs 23, similar to the prongs 14, and is substantially coplanar with the outer surface of the rest of the head-plate. When the metal bar of the hinge portion is present in the extension and hole 22 then correspondingly the inner surface of the hinge portion and the inner surface of the head-plate are also substantially coplanar.

If desired, the extension 21 may terminate in a tongue 24 formed with a hole 25 through which a peg or screw may be passed into an unthreaded or threaded hole in the metal bar so as to secure the head-plate to the metal bar of the hinge portion.

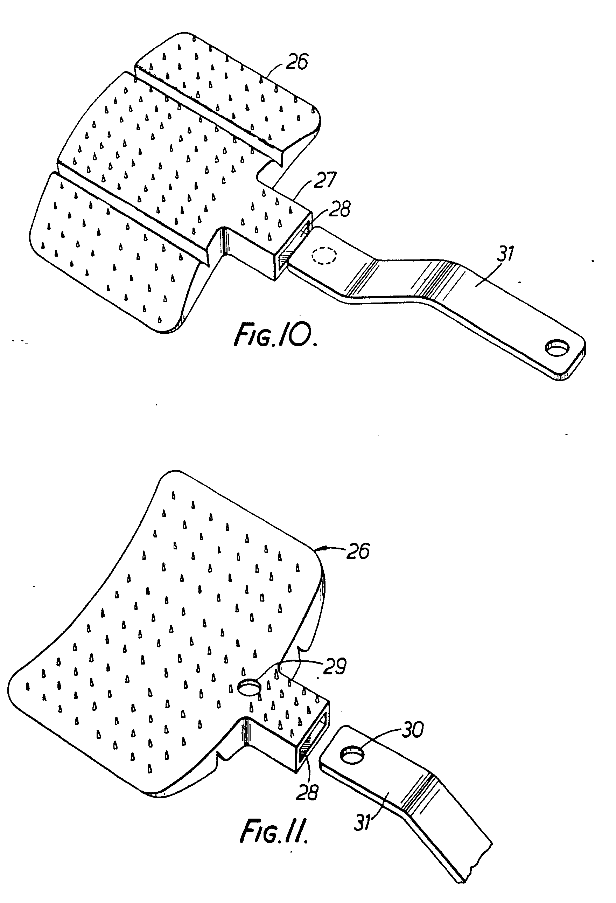

The head-plate 26 shown in Figures 10 and 11 is similar to that shown in Figures 8 and 9 but the extension 27 is a hollow box-shaped extension into which the free end of the hinge arm can be fitted in a slot 28. It will be noted that the head-plate 26 and the extension 27 have substantially continuous outer and inner surfaces, the slot 28 being formed within the extension. A hole 29 is formed in the inner surface of the head-plate 26 to receive a pip 30 on the end of an arm 31 of a hinge portion when the arm is pushed fully home into the slot thereby to provide firm engagement of the arm and positive capture of the arm in the slot.

The slot 28 is so dimensioned as to fit the hinge arm closely on all five contact surfaces and the central region is extended so far that a substantial part of the arm 31 is contained in the slot, the edges of which are tapered towards the arm. The extension 27 is entirely incorporated into the cast. Removal of the hinge arm from the head-plate and therefore from the cast is effected by pulling the arm out when the thin inner layer of the cast and the flexibility of the base of the slot allows the pip to disengage from the hole therein. The head-plates are disposed of with the cast and in order to re-use the hinges, new head-plates are simply push fitted to them. It will readily be appreciated that although the method of use shown employs a single pip capture, more pips and holes could be used. Similarly, the capture could alternatively be in the form of a small rectangular key to engage with a rectangular hole. This and a variety of other similar capture configurations are contemplated.

Commercial metal hinges such as those available for use with the head-plates shown in Figures 8 to 11 are supplied with planar hinge arms;

that is to say there is no offset in them. Typically, the arms of each hinge are of unequal length but the corresponding arms in a pair of hinges are the same length, hence the overall length of both hinges is always the same. Offsetting has to be done on site by whoever fits the hinges to the patient, and this may be done by nurses as well as plaster technicians and orthopaedic technicians. Manufacturers of present metal hinges supply crude bending irons so that the arms can be shaped as required. However, it is very difficult to work accurately with these and considerable physical strength is needed to achieve bending. We have encountered numerous instances of this being beyond the strength of nurses called upon to attempt it and many examples in British hospitals of bending irons themselves being damaged in normal use. Furthermore, there is a risk of damage to the hinge mechanism itself during the bending process.

Another consequence of on-site shaping of hinge arms is the waste of time caused in busy plaster rooms. Each hinge arm has to be bent away from its own plane and outwards to achieve an offset. It is then bent again back into a plane parallel to the original plane. This is two bends per arm, eight in all for a pair of hinges. The procedure takes several minutes.

It is normal for the medial condyle of a patient to protrude to a greater extent than the lateral condyle. This effect is especially marked in fat patients where thick tissue overburden requires greater offset to be formed in the hinge if adequate medial condylar clearance is to be obtained. Clearly the overall length of a hinge arm is the sum of its offset and non-offset lengths and this will always be more than the effective length of the hinge in the non-offset plane. Therefore when a pair of condylar brace hinges of equal length are formed with offsets of different depths to obtain the required condylar clearance, the result is that the two hinges are of different effective lengths in the non-offset plane.

When compensating for a considerable degree of medial condylar protuberance in large or fat patients the resulting shortening of the medial hinge can be considerable. One of the consequences of the shortening and different overall lengths of the hinges is an increase in the amount of cast bandage used to bind-in the hinges to the casts. This is due to the head-plates falling at different positions on the inner and outer aspects of the limb requiring separate cast bandaging to be used to ensure that each of the head-plates is adequately secured.

Almost all authorities consider the use of an alignment jig mandatory for existing metal cast brace hinges. Such a jig is intended to align the hinge axes of medial and lateral hinges with the flexion axis of the knee. Provided that the actual offset formed at each end of a hinge is identical, the axes will align and remain aligned throughout flexion. However, if the offsets are not identical at each end of the hinge, which condition is virtually certain to obtain when manual bending with irons is employed, the hinge axes may well lie on the knee axis but they will not be coaxial. This is because any hinge with different offsets at each end will lie at an angle. Such a condition will result in torsional forces being imparted to the casts and possibly to the limb, thus defeating one object of cast bracing — the prevention of rotation. The size and effect of torsional forces will vary according to the linear difference between the offsets. The effect is compounded if, as often happens, a twist is imparted to hinge arms during bending. This problem is well known to those skilled in the art of cast bracing and necessitates the building out of the cast to compensate. This is wasteful of both time and material.

In practice, it is impossible, using current bending irons which are similar in appearance and construction to motor car and bicycle tyre levers, to achieve the degree of accuracy which the use of an alignment jig demands. Current levers are hand held in one hand and are usually used with the hinge held in the other hand. Given the design of bending irons this is inherently inaccurate. We have shown that, even with the one end of a metal hinge held in a bench mounted vice, it is very difficult to achieve accurate bending. Several attempts may be necessary during which the hinge arm metal becomes work hardened and sometimes partly fatigued.

The bending irons themselves sustain damage with relatively little normal use and they impart damage to the hinge metal as a result of surface drag and stretch. Prior art hinges also sustain damage during removal from a cast — a procedure referred to as "chiselling out".

Commercially supplied cast brace hinges are generally intended to be discarded after a single use which reflects an awareness by suppliers of the amount of damage they receive during shaping and during chiselling out of the head-plates and parts of the hinge arms from the casts.

Overall, the fact that the commercially available metal hinges have to be formed by hand minimises the benefits of using an alignment jig and leads to hinge damage and a waste of time. The absence of a simple mechanism for detaching head-plates from hinge arms after a cast is removed has made the recovery of hinges already damaged by crude bending, marginal.

Referring now to Figures 12 and 13, there is shown a pair of cast bracing hinges for fracture casts. Head-plates which may be those shown in Figures 8 and 9 or Figures 10 and 11 are not shown for the sake of clarity. The bracing hinge shown in Figure 12 is a lateral hinge and comprises two hinge arms 31 and 32 each formed with an offset and connected together at the offset ends. The offset is such that the offset portions of the hinge arms lie in a plane spaced a distance $y$ from the plane in which the free ends of the hinge arms lie. The offset end of each arm is formed with a hole and fixing screws 35 and 36 pass through the holes and secure the arms to brackets 37 and 38 mounted above and below the ends thereby forming a bipivotal hinge.

The bracing hinge shown in Figure 13 is a medial hinge and is similar to the hinge shown in Figure 12. It has two hinge arms 33 and 34, each formed with an offset and connected together at the offset ends by connecting means 35 to 38. The difference between the hinge shown in Figure 13 and that shown in Figure 12 is that the offset distance $x$ is greater than the offset distance $y$ so as to allow greater clearance for the medial condyle. The arms 33 and 34 are slightly longer than the arms 31 and 32, in the unformed condition, so as to allow for the greater offset distance and to ensure that the overall length $l$ of each finished bracing hinge is the same.

The free ends of the arms 31 to 34 are formed with holes or other means whereby they can be attached to head-plates, for example the pip 30 described with reference to Figures 10 and 11.

The hinge arms are made of steel or aluminium alloy and the offsets are formed accurately in press tools and not inaccurately on site.

It will be appreciated that the bracing hinges shown in Figures 12 and 13 will be sold or made available as a pair and that many modifications of the bracing hinges are possible. The hinge is shown as bipivotal but it will be appreciated that it could be a monocentric or polycentric arrangement.

It will be appreciated that because of the good capture of the head-plates of the present hinge-brace in the cast, it becomes feasible to attach the hinge arms to the head-plates in such a way that they can be detached therefrom when the cast is removed and discarded and therefore the recovery of accurately formed hinge arms becomes a practical proposition.

As indicated above, a previously proposed hinge brace is in the form of a thin rectangular head-plate to which the end of the hinge portion is riveted and thus stands up proud above the head-plate. In contradistinction, the present hinge brace is so shaped as to enable a flush fitting arrangement of the head-plate and hinge portion to be achieved thereby eliminating dead spaces and considerably improving lamination, as well as reducing the possibility of relative motion occurring between the hinge portion and the head-plate.

The present invention makes it possible to design the head-plate so that it will be thinner at its thickest central portion than the thickness of a comparable previously proposed hinge brace.

Furthermore, due to the improved lamination which can be achieved with the present hinge brace, the bond strengths which can be obtained using modern resin casting materials may equal or surpass those which can be obtained using Plaster of Paris, which may, itself, of course, be used with the present hinge brace.

## Claims

1. A hinge-brace for a fracture cast comprising a head-plate for incorporation in a fracture cast, the head-plate having inner and outer surfaces with respect to a limb to which it is to be applied, and being secured to one end of a hinge portion, characterised in that the hinge brace comprises at least one head-plate (2, 12, 20, 26), the central region of the, or each of which is thicker than the edge regions, the central region or an extension of the head-plate being formed with a recess (3, 8, 22, 28) and the end of the hinge portion being shaped in a manner complementary to said recess and being a close fit therein, and in that the inner and outer surfaces of the hinge-brace which are intended to lie within the cast are bounded by substantially smooth concave and convex surfaces respectively, whereby when the head-plate is embedded in casting material, the formation of dead spaces in the region where the hinge portion joins the head-plate is substantially eliminated.

2. A hinge-brace as claimed in Claim 1, wherein the hinge portion is a polycentric hinge portion (1, 11).

3. A hinge-brace as claimed in Claim 1, wherein the hinge portion is a monocentric hinge portion.

4. A hinge-brace as claimed in any one of Claims 1 to 3, wherein the hinge portion is made of plastics.

5. A hinge-brace as claimed in any one of Claims 1 to 3, wherein the hinge portion is made of metal.

6. A hinge-brace as claimed in any one of Claims 1 to 5, wherein a head-plate is secured at each end of the hinge portion.

7. A hinge-brace as claimed in any one of Claims 1 to 6, wherein the hinge portion and head-plate are so shaped and secured to one another that the outer surface of the head-plate and a corresponding outer surface of the hinge portion are substantially coplanar and that the inner surface of the head-plate and a corresponding inner surface of the hinge portion are likewise substantially coplanar, said outer and inner coplanar surfaces being those which are intended to lie within the cast.

8. A hinge-brace as claimed in any one of Claims 1 to 6, wherein the or each head-plate is formed with an extension (21, 27) from said thicker central region for receiving the end of a hinge portion (31), the extension having at least one surface coplanar with a corresponding surface of the head-plate and intended to lie within the cast.

9. A hinge-brace as claimed in any one of Claims 1 to 7, wherein the head-plate has substantially continuous outer and inner surfaces and said recess is a slot formed within the central region.

10. A hinge-brace as claimed in Claim 8, wherein the head-plate and extension have substantially continuous outer and inner surfaces, and said recess is a slot formed within said extension.

11. A hinge-brace as claimed in Claim 7, wherein the head-plate has an open recess in one of said surfaces and the end of the hinge portion is rebated to fit closely in said recess and to provide the substantially coplanar surfaces.

12. A hinge-brace as claimed in any preceding claim, wherein the head-plate is arcuate crescent-shaped in cross-section.

13. A hinge-brace as claimed in any preceding claim, wherein at least one of the outer and inner surfaces of the hinge-brace intended to lie within the cast is provided with means (9, 13, 14, 15) for promoting mechanical engagement or capture between the hinge-brace and the casting material.

14. A hinge-brace as claimed in Claim 13, wherein said means comprises teeth or splines (9) formed on one or both of said surfaces.

15. A hinge-brace as claimed in Claim 14, wherein said teeth or splines are arranged on the or each surface in two groups, and wherein the teeth or splines of one group extend in a different direction from those of another group.

16. A hinge-brace as claimed in Claim 13, wherein said means comprises prongs (13, 14, 15) formed on one or both of said surfaces.

17. A hinge-brace as claimed in Claim 16, wherein the outer surface of the hinge-brace is formed with prongs and the inner surface is formed with prongs which are shorter than those formed on the outer surface.

18. A hinge-brace as claimed in any preceding claim, wherein the outer and inner surfaces intended to lie within the cast are roughened.

19. A hinge-brace as claimed in any preceding claim, wherein the head-plate is formed with two parallel grooves (17) on either side of said region where the hinge portion joins the head-plate, and wherein the head-plate can be bent along said grooves to conform more closely to the limb to be braced.

20. A hinge-brace as claimed in any preceding claim, wherein the or each head-plate is made of plastics material.

21. A hinge-brace as claimed in any preceding claim, wherein the or each head-plate is made of polyethylene or polypropylene and the hinge portion is a polycentric hinge portion made of nylon or polycarbonate.

22. A pair of the hinge-braces claimed in any preceding claim, wherein each hinge-brace comprises a hinge portion having two free end portions (31, 32; 33, 34) for receiving head-plates substantially lying in a first plane and an intermediate portion incorporating hinge means (35, 36, 37, 38) and substantially lying in a second plane offset from the first, and wherein the depth of offset of one hinge portion adapted to constitute a medial hinge is greater than the depth of offset of the other hinge portion adapted to constitute a lateral hinge, the hinge portions being of substantially the same overall length.

23. A pair of hinge-braces as claimed in Claim 22, wherein each hinge portion comprises two hinge arms each formed with an offset portion with the ends of the offset portions connected by hinge means, the free ends of the arms lying in said first plane and the connected ends lying in said second plane.

**Patentansprüche**

1. Gelenkschiene für einen Knochenbruch-Einbettungverband, umfassend eine Kopfplatte für den Einbau in einen Knochenbruch-Einbettungsverbund, wobei die Kopfplatte Innen- und Außenflächen in Bezug auf ein Körperglied, für das sie verwandt wird, besitzt und sie an einem Ende eines Gelenkteiles befestigt ist, dadurch gekennzeichnet, daß die Gelenkschiene wenigstens eine Kopfplatte (2, 12, 20, 26) umfaßt, deren Zentralbereich(e) dicker ist (sind) als die Randbereiche, wobei der Zentralbereich oder ein Fortsatz der Kopfplatte mit einer Vertiefung (3, 8, 22, 28) ausgebildet ist und das Ende des Gelenkteiles derart gestaltet ist, daß sie komplementär zu besagter Vertiefung ist und darin formschlüssig eingepaßt ist,

und dadurch, daß die Innen- und Außenflächen der Gelenkschiene, die innerhalb des Einbettungsverbandes zu liegen kommen sollen, durch im wesentlichen glatte konkave bzw. konvexe Oberflächen begrenzt sind, wobei dann, wenn die Kopfplatte in Einbettungsmaterial eingebettet ist, die Ausbildung von Hohlräumen in dem Bereich im wesentlichen ausgeschlossen ist, wo das Gelenkteil mit der Kopfplatte verbunden ist.

2. Gelenkschiene nach Anspruch 1, dadurch gekennzeichnet, daß der Gelenkteil ein polyzentrischer Gelenkteil (1. 11) ist.

3. Gelenkschiene nach Anspruch 1, dadurch gekennzeichnet, daß der Gelenkteil ein monozentrischer Gelenkteil ist.

4. Gelenkschiene nach einem der Ansprüche 1 bis 3, dadurch gekennzeichnet, daß der Gelenkteil aus Kunststoff hergestellt ist.

5. Gelenkschiene nach einem der Ansprüche 1 bis 3, dadurch gekennzeichnet, daß der Gelenkteil aus Metall hergestellt ist.

6. Gelenkschiene nach einem der Ansprüche 1 bis 5, dadurch gekennzeichnet, daß eine Kopfplatte an beiden Enden des Gelenkteiles befestigt ist.

7. Gelenkschiene nach einem der Ansprüche 1 bis 6, dadurch gekennzeichnet, daß der Gelenkteil und die Kopfplatte so geformt und aneinander befestigt sind, daß die Außenfläche der Kopfplatte und eine entsprechende Außenfläche des Gelenkteiles im wesentlichen koplanar sind und daß die Innenfläche der Kopfplatte und eine entsprechende Innenfläche des Gelenkteiles ebenfalls im wesentlichen koplanar sind, wobei die äußeren und inneren koplanaren Oberflächen solche sind, bei denen vorgesehen ist, daß sie innerhalb der Einbettung liegen.

8. Gelenkschiene nach einem der Ansprüche 1 bis 6, dadurch gekennzeichnet, daß die Kopfplatte (n) mit einem Fortsatz (21, 27) ausgehend vom genannten dickeren Zentralbereich ausgebildet ist (sind), der das Ende eines Gelenkteils (31) aufzunehmen, wobei der Fortsatz wenigstens eine Oberfläche hat, die mit einer entsprechenden Oberfläche der Kopfplatte koplanar ist und bei der vorgesehen ist, daß sie innerhalb der Einbettung zu liegt.

9. Gelenkschiene nach einem der Ansprüche 1 bis 7, dadurch gekennzeichnet, daß die Kopfplatte im wesentlichen durchgehende Außen- und Innenflächen hat und daß die genannte Vertiefung als ein innerhalb des Zentralbereiches ausgebildeter Schlitz ist.

10. Gelenkschiene nach Anspruch 8, dadurch gekennzeichnet, daß die Kopfplatte und der Fortsatz im wesentlichen durchgehende Auben- und Innenflächen besitzt und daß die genannte Vertiefung ein innerhalb des genannten Fortsatzes ausgebildeter Schlitz ist.

11. Gelenkschiene nach Anspruch 7, dadurch gekennzeichnet, daß die Kopfplatte eine offene Vertiefung in einer der genannten Oberflächen besitzt und daß das Ende des Gelenkteils zurückgenommen ist, um formschlüssig in der genannten Vertiefung eingepaßt werden zu können und um die im wesentlichen koplanaren Oberflächen zu liefern.

12. Gelenkschiene nach einem der vorherigen Ansprüche, dadurch gekennzeichnet, daß die Kopfplatte im Querschnitt halbmondförmig gebogen ist.

13. Gelenkschiene nach einem der vorherigen Ansprüche, dadurch gekennzeichnet, daß wenigstens eine der Außen- und Innenflächen der Gelenkschiene, bei der vorgesehen ist, daß sie innerhalb der Einbettung liegen, mit Mitteln (9, 13, 14, 15) versehen ist, um mechanisches Festhaken oder Aneinanderhaften zwischen Gelenkschiene und Einbettungsmaterial zu fördern.

14. Gelenkschiene nach Anspruch 13, dadurch gekennzeichnet, daß die genannten Mittel Zähne oder Keilnuten (9) umfassen, die auf einer oder auf beiden Seiten der genannten Oberflächen ausgebildet sind.

15. Gelenkschiene nach Anspruch 14, dadurch gekennzeichnet, daß die genannten Zähne oder Keilnuten auf der oder auf jeder Oberfläche in zwei Gruppen angeordnet sind und daß die Zähne oder Keilnuten der einen Gruppe in einer anderen Richtung als die der anderen Gruppe ausgerichtet sind.

16. Gelenkschiene nach Anspruch 13, dadurch gekennzeichnet, daß die Mittel Zacken (13, 14, 15) umfassen, die auf einer oder auf beiden der genannten Oberflächen ausgebildet sind.

17. Gelenkschiene nach Anspruch 16, dadurch gekennzeichnet, daß die Außenfläche der Gelenkschiene Zacken besitzt und die Innenfläche mit Zacken versehen ist, die kürzer als die Zacken auf der Außenfläche sind.

18. Gelenkschiene nach einem der vorherigen Ansprüche, dadurch gekennzeichnet, daß die Außen- und die Innenflächen, die innerhalb der Einbettung zu liegen kommen, aufgerauht sind.

19. Gelenkschiene nach einem der vorherigen Ansprüche, dadurch gekennzeichnet, daß die Kopfplatte mit zwei parallelen Nuten (17) auf jeder Seite des Bereiches ausgebildet ist, wo das Gelenkteil mit der Kopfplatte verbunden ist, und daß die Kopfplatte entlang der genannten Nuten biegsam ist, um näher zu dem abzustützenden Körperglied ausgerichtet werden zu können.

20. Gelenkschiene nach einem der vorherigen Ansprüche, dadurch gekennzeichnet, daß die Kopfplatte(n) aus Kunststoffmaterial hergestellt ist (sind).

21. Gelenkschiene nach einem der vorherigen Ansprüche, dadurch gekennzeichnet, daß die Kopfplatte(n) aus Polyethylen oder Polypropylen hergestellt ist (sind) und daß das Gelenkteil ein polyzentrisches Gelenkteil ist, das aus Nylon oder Polykarbonat hergestellt ist.

22. Ein Paar Gelenkschienen nach einem der vorherigen Ansprüche, dadurch gekennzeichnet, daß jede Gelenkschiene einen Gelenkbereich umfaßt, der zwei freie Endteile (31, 32; 33, 34) besitzt, um die Kopfplatten aufzunehmen, wobei die Endteile im wesentlichen in einer ersten Ebene angeordnet sind,

und dadurch, daß ein Zwischenstück vorhanden ist, das die Gelenkmittel (35, 36, 37, 38) umfaßt und im wesentlichen in einer zweiten von der ersten abgesetzten Ebene angeordnet ist, wobei das Maß der Absetzungsebene des einen Gelenkteiles, das als mediales mediales Gelenk dient, größer ist als das Maß der Absetzung des anderen Gelenkteiles, das als ein laterales Gelenk dient, wobei die Gelenkteile im wesentlichen dieselbe Gesamtlänge haben.

23. Ein Paar Gelenkschienen nach Anspruch 22, dadurch gekennzeichnet, daß jedes Gelenkteil zwei Gelenkarme umfaßt, die jeder mit einem abgesetzten Teil ausgebildet ist, das mit den Enden der Stufenteile durch Gelenke verbunden ist, wobei die freien Enden der Arme in der genannten ersten Ebene und die verbundenen Enden in der genannten zweiten Ebene liegen.

**Revendications**

1. Armature à charnière destinée à un plâtre orthopédique comprenant une plaque de tête incorporée dans un plâtre orthopédique, la plaque de tête ayant des surfaces intérieure et extérieure par rapport à une membre auquel elle doit être appliquée, et étant fixée à une extrêmité d'une partie charnière, caractérisée en ce que l'armature à charnière comprend au moins une plaque de tête (2, 12, 20, 26), dont la zone centrale ou les zones centrales sont plus épaisses que les zones marginales, la zone centrale ou un prolongement de la plaque de tête comprenant un renfoncement (3, 8, 22, 28) et l'extrêmité de la partie charnière étant formée de façon complémentaire au renfoncement et y étant ajustée étroitement serrée, et en ce que les surfaces intérieure et extérieure de l'armature à charnière qui doivent se trouver à l'intérieur du plâtre, sont liées par des surfaces relativement lisses concave et convexe respectivement, ce qui permet, lorsque la plaque de tête est encastrée dans la matière de moulage, d'éliminer sensiblement la formation d'espaces nuisibles dans la zone où la partie charnière est réunie à plaque de tête.

2. Armature à charnière selon revendication 1, dans laquelle la partie charnière est une charnière polycentrique (1, 11).

3. Armature à charnière selon revendication 1, dans laquelle la partie charnière est une charnière monocentrique.

4. Armature à charnière selon l'une quelconque des revendications 1 à 3, dans laquelle la partie charnière est réalisée en plastique.

5. Armature à charnière selon l'une quelconque des revendications 1 à 3, dans laquelle la partie charnière est réalisée en métal.

6. Armature à charnière selon l'une quelconque des revendications 1 à 5, dans laquelle la plaque de tête est fixée à chaque extrémité de la partie charnière.

7. Armature à charnière selon l'une quelconque des revendications 1 à 6, dans laquelle la partie charnière et la plaque de tête sont façonnées et fixées l'une à l'autre de telle façon que la surface extérieure de la plaque de tête et une surface extérieure correspondante de la partie charnière soient sensiblement coplanaires et que la surface intérieure de la plaque de tête et une surface intérieure correspondante de la partie charnière soient également sensiblement coplanaires, ces surfaces coplanaires extérieures et intérieures étant celles devant se trouver à l'intérieur de plâtre.

8. Armature à charnière selon l'une quelconque des revendications 1 à 6, dans laquelle la plaque de tête ou chacune des plaques de tête est pourvue d'un prolongement (21, 27) s'étend depuis la zone centrale plus épaisse pour recevoir l'extrémité d'une partie charnière (31), le prolongement ayant au moins une surface coplanaire avec une surface correspondante de la plaque de tête et devant se trouver à l'intérieur du plâtre.

9. Armature à charnière selon l'une quelconque des revendications 1 à 7, dans laquelle la plaque de tête a des surfaces extérieure et intérieure relativement continues et le renfoncement est une fente formée dans la region centrale.

10. Armature à charnière selon revendication 8, dans laquelle la plaque de tête et le prolongement ont des surfaces extérieure et intérieure relativement continues et le renfoncement est une fente formée à l'intérieur du prolongement.

11. Armature à charnière selon revendication 7 dans laquelle la plaque de tête possède un renfoncement ouvert dans l'une de ces surfaces et l'extrêmité de la partie charnière est entaillée pour être ajustée étroitement dans le renfoncement et assurer les surfaces sensiblement coplanaires.

12. Armature à charnière selon l'une quelconque des revendications précédentes dans laquelle la plaque de tête est en forme de demi-lune arquée sur sa coupe transversale.

13. Armature à charnière selon l'une quelconque des revendications précédentes, dans laquelle au moins l'une des surfaces extérieure et intérieure de l'armature à charnière devant se trouver à l'intérieur du plâtre, est pourvue d'un moyen (9, 13, 14, 15) pour provoquer la liaison mécanique entre l'armature de charnière et la matière de moulage.

14. Armature à charnière selon revendication 13, dans laquelle ce moyen comprend les dents ou clavettes (9) formées sur l'une ou sur les deux surfaces.

15. Armature à charnière selon revendication 14, dans laquelle les dents ou clavettes sont disposées sur la surface ou sur chacune des surfaces en deux groupes, et dans laquelle les dents ou clavettes d'un groupe s'étendent dans un sens différent de ceux d'un autre groupe.

16. Armature à charnière selon revendication 13, dans laquelle le moyen comprend les dents (13, 14, 15) formées sur l'une ou sur les deux surfaces.

17. Armature à charnière selon revendications 16, dans laquelle la surface extérieure de l'armature à charnière est pourvue de dents et la surface intérieure comprend des dents plus courtes que celles formées sur la surface extérieure.

18. Armature à charnière selon l'une quelconque des revendications précédentes dans laquelle les surfaces extérieure et intérieur devant se trouver à l'intérieur du plâtre, sont rendues rugueuses.

19. Armature à charnière selon l'une quelconque des revendications précédentes, dans laquelle la plaque de tête est pourvue de deux rainures parallèles (17) de chaque côté de la zone où la partie charnière est réunie à la plaque de tête, et dans laquelle la plaque de tête peut être pliée le long des rainures pour l'adapter plus étroitement au membre devant être armaturé.

20. Armature à charnière selon l'une quelconque des revendications précédentes dans laquelle la plaque de tête ou chaque plaque de tête est réalisée en plastique.

21. Armature à charnière selon l'une quelconque des revendications précédentes dans laquelle la plaque de tête ou chaque plaque de tête est réalisée en polyéthylène ou polypropylène et la partie charnière est une charnière polycentrique fabriquée en nylon ou polycarbonate.

22. Deux armatures à charnière selon l'une quelconque des revendications précédentes, dans lesquelles chaque armature à charnière comprend un partie charnière ayant deux extrêmités libres (31, 32; 33, 34) pour recevoir les plaques de tête se trouvant sensiblement sur un premier plan et une partie intermédiaire comprenant le moyen de charnière (35, 36, 37, 38) et se trouvant sensiblement sur un second plan déporté par rapport au premier, et dans lesquelles la profondeur de déport d'une partie charnière adaptée pour constituer une charnière médiane est supérieure à la profondeur du déport de l'autre partie charnière adaptée pour constituer une charnière latérale, les parties charnières étant sensiblement d'une longueur totale identique.

23. Deux armatures à charnière selon revendication 22, dans lesquelles chaque partie charnière comprend deux bras, chacun étant pourvu d'une partie déportée et les extrêmités des parties déportées étant raccordées par le moyen de charnière, les extrêmités libres des bras étant situées sur la premier plan et les extrémités raccordées sur le second plan.

0 109 847

Fig. I.

Fig. IA

FIG. 2.

FIG. 3.

FIG. 4.

FIG. 5.

Fig.6.

Fig.7.

FIG.8.

FIG.9.

FIG.10.

FIG.11.

FIG. 12.

FIG. 13.